# EUROPEAN PATENT APPLICATION

(11) **EP 2 568 038 A1**
(43) Date of publication of application: **13.03.2013**
(21) Application number: 11777300.2
(22) Date of filing: 07.04.2011
(51) Int. Cl.: C12M 3/00

(54) **LAMINAR PHOTOBIOREACTOR FOR THE PRODUCTION OF MICROALGAE**

(30) Priority: 03.05.2010 ES 201030651
(71) Applicant: Universidad Politécnica De Madrid, 28040 Madrid (ES)
(72) Inventor: FERNÁNDEZ GONZÁLEZ, Jesús, E-28040 Madrid (ES)
(74) Representative: Carvajal y Urquijo, Isabel
(86) International application number: PCT/ES2011/000104
(87) International publication number: WO 2011/138477

(57) **Abstract**

The present invention relates to a modular photobioreactor for the production of microalgae particularly indicated for absorber emission gases with a high carbon dioxide (CO₂) content. It is based on the continuous recirculation of a microalgae-containing liquid medium through sheets of fabric facilitating CO₂ absorption and microalgae illumination. The invention enables supplying said gases to the culture from inside the chamber. It is advantageous in that it provides highly efficient algae illumination, allows easy exchange of CO₂ from the emission gases to the culture and can be used at a low cost on a large scale.

## Description

### Technical Field of the invention

The laminar photobioreactor of the invention is applicable in the field of large scale microalgae biomass production. The biomass produced can serve as a raw material for obtaining biofuels, feeds and food products, as well as for future biorefining. It can also be used as a sink for greenhouse gases, mainly carbon dioxide (CO₂) and nitrogen oxides from industrial plants with a minimum risk for operators and the environment.

### Background of the Invention

The use of microalgae for producing biomass is an old idea having its origins in the 1970's as a result of the first oil crisis in 1973, research works being conducted in various laboratories worldwide to attempt to produce liquid or gaseous biofuels. In this sense the work carried out in the United States between 1978 and 1986 which concluded even then that the production of biofuels with microalgae was potentially viable from a technical viewpoint, even though yet to be from an economic viewpoint, was very important.

In recent years the idea of producing biofuels from microalgae has resurfaced in force with a considerable increase in the publication of works dedicated to microalgae production and CO₂ capture, obtaining strains with specific characteristics or obtaining products for use in different sectors of the industry and in health. This growing interest for microalgae culture is motivated, among other reasons, by the fact that microalgae are considered as potential CO₂ sinks of industrial origin, by oil price instability and insecurity in future oil supply and by the discredit which the use of raw materials for food use faced in biofuel production among the general public.

Despite the growing interest for microalgae culture, a commercial system capable of producing microalgae biomass at competitive prices for making biofuel production viable from an economic viewpoint is yet to be achieved. To achieve a high microalgae biomass production, the following limiting factors must be controlled:
a) Suitable microalgae illumination necessary for performing photosynthesis and the energy from the visible radiation is converted into chemical energy for obtaining activated electrons, among other products.
b) Continuous supply of CO₂ during the microalgae illumination phase necessary for accepting the activated electrons and producing the starting molecules of the photosynthetic metabolism (sugars).
c) Removal of oxygen formed in photosynthesis so that the microalgae photosynthetic capacity is not affected by photorespiration.
d) Suitable temperature for the type of microalgae to be cultured (including psychrophiles, mesophiles and thermophiles).
e) Nutrients in suitable proportion and amount.
f) Physicochemical characteristics of the culture medium (mainly pH, conductivity and salinity).

The large scale microalgae culture systems used until now include two basic types, each of them with different variants: Channels or tanks open to the atmosphere with or without energy consumption for medium recirculation and photobioreactors. In the latter the microalgae-containing medium is isolated from the atmosphere and is continuously recirculated inside transparent structures made of glass or plastic material having various shapes, such as tubular coils, tubes, bags or vertical panels, panels or inclined tubes or combinations thereof.

The channels or tanks open to the atmosphere have a reasonable construction cost, however they also have a relatively low productivity due to the difficulty in providing the CO₂ necessary to the culture and the difficulty in illuminating all the algae present in the medium uniformly when the culture is growing. CO₂ is transferred by diffusion to the aqueous medium from air where the concentration of this compound is relatively low, in the order of 0.03%. In channels with forced water circulation, these effects are mitigated but the energy consumption increases the production price making it economically unviable for biofuel production.

In the photobioreactors existing until now, with the culture medium confined inside the transparent structures, factors relating to temperature, nutrients and culture medium, even the microalgae illumination can be suitably controlled by designing a suitable geometry and/or using artificial illumination, but the production cost for obtaining biofuels is not economically viable so far. The use of compressed CO₂ which is prohibitory for large scale biomass production is resorted to for increasing the concentration of CO₂ in the medium of the experimental photobioreactors. To remove the oxygen formed in photosynthesis and to prevent its inhibiting effect, open aeration systems which also involve a very high cost due to the need to restore the compressed CO₂ continuously, are used. Even though gases from industrial plant fossil fuel combustion can theoretically be used, commercial plants which are economically viable have not been set up until now.

The first tubular photobioreactors were patented at the end of last century, among them the photobioreactor developed by Dr. Otto Pulz and his team (WO1998/045409 stands out. Among the recent patents related to such photobioreactors for microalgae culture the following are included:
Application US 2009/0203116 A1 describes a tubular reactor in which the inner illumination is strengthen by means of fiber optics. US 2009/0151241 A1 describes the use of a "perfluorodecalin" solution and an emulsified surfactant for increasing the CO₂ solubility in the medium and facilitating the removal of oxygen formed in photosynthesis. In turn, US 2008/0286851 A1 describes a closed reactor, constructed with light, transparent plastic, materials which can be deployed in the field. All of them involve a relatively expensive investment. Their maintenance cost for obtaining low price products, such as the raw material for producing biofuels, is also too high.

The present invention provides a solution to the problem of algae illumination by passing microalgae over a support sheet made of geotextile, allowing a complete and simultaneous illumination of all the microalgae passing over said sheet. It also allows removing the oxygen easily since the outer face of the geotextile is exposed to the atmosphere whereas the inner face receives CO₂ by diffusion in a suitable proportion.

International application WO 2008/008262 A2 describes a system of closed linear photobioreactors formed by channels with a transparent cover through which the medium with the algae and the mixture of CO₂-enriched gases flow. The covered channels can be interconnected. WO 2008/134010 describes a closed photobioreactor with a plastic cover floating on a channel through which a stream of liquid medium with microalgae and gases from industrial plant emission flows. WO 2007/011343 A1 describes an illuminated, iriclined, tubular reactor connected with another opaque vertical tube. The microalgae are passed through the illuminated tube and the opaque tube alternately. These three prior applications relates to closed bioreactors in which the high concentration of CO₂ causes "greenhouse effect" with the subsequent heating of the culture medium and algae loss. The present invention solves this problem preventing said greenhouse effect since the microalgae and the medium are in direct contact with the atmosphere. Furthermore, the invention dissipates the heat from the thermal radiation that it receives as a result of the evaporation of part of the water from the medium.

To enable large scale microalgae biomass production and to contribute considerably to the reduction in industrial plant emissions, there is a need to develop low cost, easy to install systems which allow their installation in large areas of land. Therefore, to capture 10% CO₂ which would be produced in a conventional 100 MW power plant that works for about 8000 hours a year the emissions of which are at a rate of 781 t of CO₂ per GWhₑ produced, slightly more than 600 ha of algae culture would be necessary assuming that the CO₂ fixation occurs at a rate of 150 t/ha per year (for a production of about 80 t of raw material per ha and per year).

The problem considered in the art is therefore to obtain an industrial process for an efficient microalgae biomass production both in terms of capacity and cost. The solution provided by the present invention is a laminar photobioreactor with optimum illumination and CO₂ supply conditions achieving high algae productions with low investment and maintenance costs.

### Description of the invention

The photobioreactor of the invention for the production of microalgae is particularly intended for absorbing emission gases with high CO₂ content. It is based on the continuous recirculation of a microalgae-containing liquid medium through one or several superimposed sheets of fabric, facilitating CO₂ absorption and microalgae illumination.

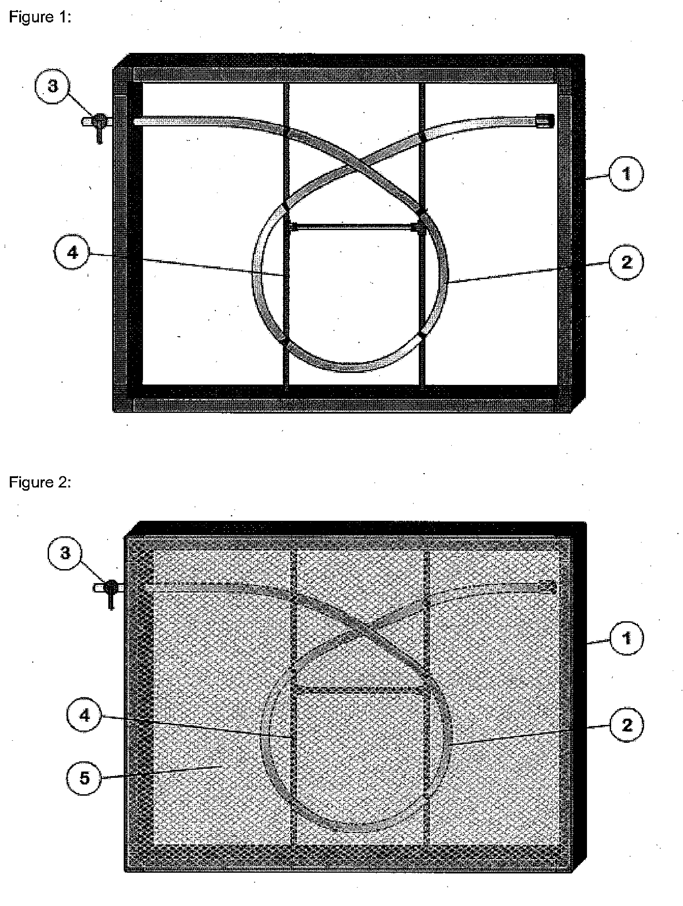

The body of each module of the photobioreactor is made up of a panel (Figures 1, 2 and 3), comprising a square or rectangular frame or rack (1) on which one or several sheets of fabric are placed on both sides. A porous pipe (2) which is connected to the outside by means of a valve (3) and which is secured to a plastic support (4) fixed to the rack of the frame is placed inside the frame. The frame with a certain thicknesses is closed on the front and rear sides with a sheet of light, mosquito net-like mesh (5). The meshes on both sides leave an air chamber demarcated by the inner faces of the rack of the frame and by the meshes themselves. The meshes must have a small enough inner open space so that the surface tension of the culture medium with microalgae causes the liquid to cover said inner open space completely and to form a continuous film as it decreases.

Sheets of fabric (6) can be placed on the sheets of mesh of the frame. Once wet, these sheets of fabric adhere directly to the sheets of mesh covering both sides of the frame and are fixed thereto by means of angular battens (7) which are adapted to the corners thereof. Therefore, different types of sheets with different properties in terms of the adherence of different microalgae can be used with the same frame-support.

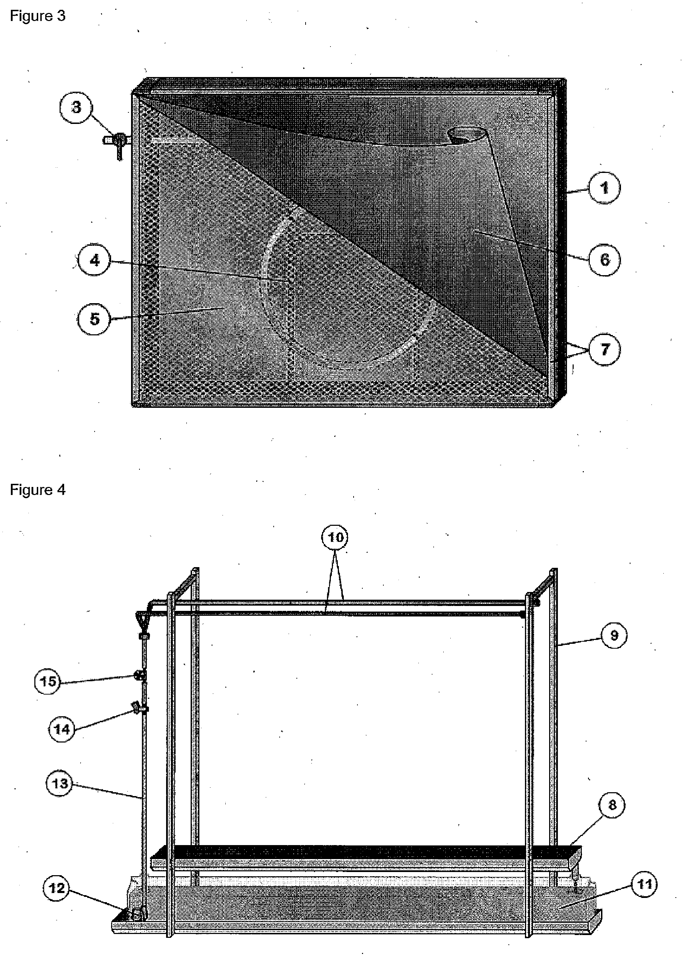

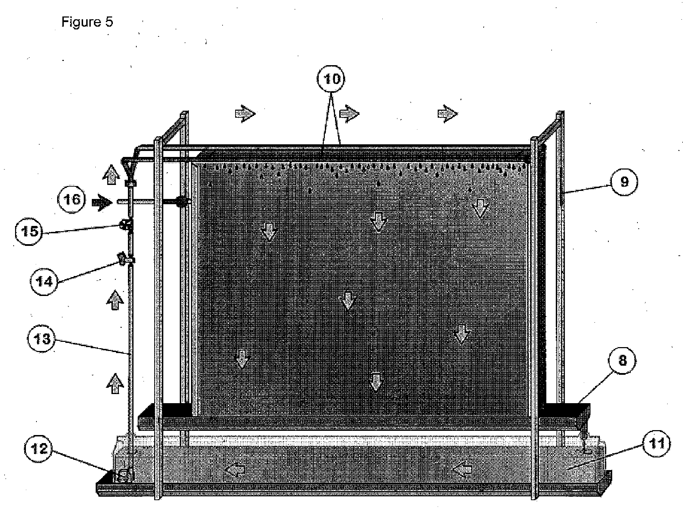

The assembly is placed in a vertical position on a supporting structure (Figures 4 and 5), having in its lower part and at a certain height from the ground, a channel (8) with a width slightly greater than that of the frame and on which the bottom part of the panel rests. The sides of the panel are fixed on the sides of the structure (9).

The invention is therefore a laminar photobioreactor for the production of microalgae, comprising a panel formed by a rack or frame covered at both sides by at least one sheet of mesh through which a microalgae-containing liquid medium flows downwards, and an inner chamber between said sheets of mesh and the walls of the rack or frame impermeable to air by means of hydraulic closure. Said chamber is limited by the walls of the frame and by the side walls of wet fabric such that the wet walls constitute the hydraulic closure for the exit of the air inside the chamber.

One embodiment of the photobioreactor of the invention is that said sheet of mesh is made of synthetic material, glass fiber, natural fiber, metal material, or combinations thereof. A preferred embodiment of the photobioreactor is that said sheet of mesh is supplemented by at least one sheet of fabric, and a yet more preferred embodiment is that said sheet of fabric is made of synthetic material, natural fibers, or combinations thereof. The most preferred embodiment is that said synthetic material is selected from the group consisting of PVC, polystyrene and polypropylene.

The sheets of fabric can also be covered on the outer part with a transparent plastic when desired. Between the sheets of fabric and the transparent plastic there is a small gap of 1 or 2 cm which will allow protecting the culture from low temperatures, where appropriate, or will also allow renewing the air by convection and driving the water vapor formed, which automatically cools the panel, important for high temperatures. Therefore, another embodiment of the invention is that the photobioreactor has a transparent plastic covering on said sheets on both sides.

On the upper part of the frame and along its length the microalgae-containing liquid culture medium which moves down the side sheets forming a continuous film is discharged by means of a perforated or corrugated pipe (10). The liquid that moves down is collected in a collector channel which discharges the medium with the microalgae on a tank system (11), from which a pump (12) can drive it again through a conduit (13) provided with a flow regulating valve (14) and a flow rate indicator (15) to the pipe of the upper part of the frame (10).

Therefore, in another embodiment of the invention the lower edges of said sheets deliver the microalgae-containing liquid medium to a channel (8) communicating with a tank system (11) located in the lower part of the photobioreactor.

In the present application, a "tank system" is understood as one or several tanks arranged in series. In the event that the photobioreactor only contemplates the recirculation of the liquid medium, a single tank will be necessary in the lower part of the panel with the circulation pump. In the event that the sedimentation of the microalgae is envisaged, at least one decanting tank will be included prior to the preceding one, directly collecting the discharge from the liquid with microalgae and allowing sedimentation. The remaining liquid from said decanting tank will be collected through the next tank which will contain the circulation pump.

Therefore, a preferred embodiment of the photobioreactor of the invention is that the liquid medium is recirculated to the upper part of the frame from the tank system, and in another embodiment said tank system includes a decanting tank allowing the sedimentation of the microalgae. Another embodiment contemplates that said sedimentation occurs in the presence of flocculants.

In the outer part of the frame there is a conduit with a valve (16) connecting with the perforated or porous pipe inside the panel (2). One embodiment of the photobioreactor of the invention is that a gas is introduced into said chamber through said pipe. A preferred embodiment is that said gas is isolated from direct solar radiation. The preferred composition of said gas is air, carbon dioxide, combustion gases or the mixtures thereof.

The transfer of CO₂ to the microalgae culture medium occurs by diffusion from inside the chamber to the walls thereof, through which the culture medium flows downwards. The outer part of the side sheets is in direct contact with the atmospheric air.

The direct exposure of the sheets of fabric to the ambient air favors continuous water evaporation from the medium covering said sheets and concentrates the microalgae in the medium leading to an energy saving when separating the microalgae from the liquid phase. In the event that water evaporation from the medium is not desired, a transparent plastic film made of polyethylene can be placed, for example, adhered to the outer part of the sheets of the photobioreactor continuously along the upper part of the perforated pipe located above the frame to further prevent monospecific culture contamination, the photobioreactor thus acts isolated from the outside air.

The photobioreactor (PBR) of the invention can be used to produce algae biomass and also allows concentrating the microalgae biomass and facilitating microalgae harvesting. The following modes of operation can be considered to complement and describe the preceding information:
A) as a single-phase PBR for the production of microalgae in the liquid medium working discontinuously. For this mode, the sheets of mesh of the PBR manufactured from material with little or zero microalgae adherence such as wires mesh, PVC mesh or glass fiber, for example, are used. Once the suitable concentration of microalgae in the medium is achieved, the PBR can continue to operate for many days without replacing the evaporated water; the microalgae in the liquid medium are thus concentrated. When side meshes sides having little or zero adhesion capacity are used, the device also serves as a microalgae concentrator in the liquid medium itself. Once the concentrated algae are harvested, new medium is added to the lower tank system (11) of the photobioreactor and a new cycle is started.
B) as a single-phase PBR for the production of microalgae in the liquid medium working continuously. For this mode, the meshes of the PBR similar to that in type A) are used but the circulation of the microalgae can be stopped at will, especially at night where there is a lack of illumination. During the circulation pump shut down periods, the medium with the grown algae and the sedimentation algae can be removed from the container without affecting the microalgae retained in the liquid absorbed in the meshes of the photobioreactor. The container is then filled with a new culture medium until reaching the desired volume.
C) as a PBR with cells immobilized and adhered to the fabric that proliferate "in *situ",* being trapped until forming a thick enough layer so as to enable removing it along with the supporting fabric. For this purpose, fabrics made of geotextile type polystyrene or fabrics made of cellulose or natural fibers which would be placed on the sheets of mesh of the frames, are recommended. The supply of the medium to the perforated pipe would be stopped once the microalgae have reached the suitable density on the supporting fabric and once the sheets are air dried, the fabric with the dried adhered microalgae would be removed to be subjected to a suitable extraction process according to the products desired.
D) as a PBR with immobilized, continuously growing cells collected by decantation. It is a system similar to that described in C) but the cells are left to grow until the microalgae layer is so thick that it detaches itself and falls with the liquid medium to the collector channel. For this case intercalating a decanting tank before the tank containing the circulation pump is necessary to gradually remove the decanted microalgae masses.
E) as a mixed PBR with heterogeneous microalgae populations in which some species can be immobilized and other smaller species or species with flagella or cilia can move in the water stream without being trapped in the fabric of the sheet. For this purpose, sheets of fabric like those used in type C) would be used superimposed on the meshes. For collecting both types of algae, the circulation of the medium must be stopped and once dried, the sheets with the immobilized microalgae would be removed in order to concentrate the microalgae included in the liquid medium by circulating this over the meshes of the PBR.

The photobioreactor of the invention has the following advantages:
- a) it can be used at a low cost on a large scale,
- b) it achieves a highly efficient algae illumination,
- c) it allows easy exchange of CO₂ from the emission gases to the culture,
- d) it provides the possibility of performing the sedimentation of the microalgae of the liquid medium without affecting those which are embedded in the geotextile sheets which will continue to multiply,
- e) it enables collecting the dried microalgae included in a sheet of fabric,
- f) it allows concentrating the algae culture, and
- g) it prevents the heating of the culture because it is a device with a continuous natural cooling that removes latent heat of the water evaporated from the outer most layer of the sheet of fabric, and because the CO₂ is confined inside the chamber that is hardly reached by infrared radiation.

A PBR can be made up of several modules discharging into a common channel and receiving the culture medium with microalgae through an also common conduit, from which a connection for each panel exits. It would be a modular device due to the repetition of the same structure for an indefinite number of times having a variable length depending on the modules which are connected. Therefore, the most preferred embodiment of the invention is a set of at least two photobioreactors connected in parallel in a modular structure.

The following examples are provided in order to show the present invention in an illustrative and by no means limiting manner.

### Examples

### Example 1: Modular photobioreactor

A frame (Figure 1) was constructed with a square, hollow, PVC tube (1) having an outer section of 7 x 7 cm and a thickness of 2 mm. The frame had outer measurements of 200 cm in length, 150 cm in height and 7 cm in thickness. The inner area demarcated by the rack of the frame had a length of 1.86 cm, a height of 1.36 cm and a depth of 7 cm, which resulted in an internal volume of 177 liters. A pipe made of porous rubber (2) the pores of which open at an internal pressure starting from 0.5 atmospheres was placed inside the frame connected to the outside through the rack of the frame (3) for coupling to the pipe carrying the CO₂-enriched air. The porous pipe was placed on an H-shaped support manufactured with a PVC tube (4). A sheet of "mosquito net"-like plastic mesh having 1-square millimeter inner open space was placed on both sides of the frame, which was tensed and adhered to the latter with glue (5 in Figure 2). Both sides of the frame were coated with a 150 g/m² geotextile cloth (6 in Figure 3) which was placed on the mesh and fixed by means of 25 x 25 mm angular PVC battens (7) placed on the outer corners of the side tubes forming the walls of said frame. The wet geotextile fabric adhered perfectly to the sheet of mosquito net mesh.

The frame was placed resting at the bottom of a 10 cm wide PVC channel (8 in Figure 4) included in a steel structure (9) protected against oxidation to which it was fixed (Figure 5).

In the upper part of the sheets there was placed a corrugated pipe (10) according to a generatrix, through which the culture medium with microalgae flows. The liquid draining off the walls of the photobioreactor is collected in channel (8) and is discharged to a transparent plastic lower collector tank (11), located below the channel, the inside of which includes a submerged pump (12) moving the water up through a vertical pipe (13) to the corrugated discharge pipe (10) irrigating the top part of the frame. An intermediate valve (14) in the upward tube regulates the suitable discharge flow of the medium without causing fluid excesses which cannot be absorbed by the geotextile or a lack of flow that leaves the sheets dry. A flow indicator (15) is intercalated in the pipe to verify the movement of the liquid therein. CO₂-enriched air which is non-toxic for the algae is injected through the outer connector (16) of the porous pipe (2) inside the panel.

### Example 2: Operation of a modular photobioreactor in the "discontinuous production" mode

The photobioreactor of Example 1 with the frame covered only by the sheet of polyethylene mosquito net mesh was used for the production of algae biomass. An amount of 120 liters of culture medium was prepared using mineral compounds manufactured from fertilizers used in organic irrigation, with a molar concentration of 8.8 x 10⁻⁴ M of NO₃⁻ and 3.6 x 10⁻⁵ M of PO₄³⁻, ideal for culturing microalgae. This culture medium was introduced in the lower tank (11) of the photobioreactor. A polyspecific culture of microalgae with the predominance of *Chlorella sorokiniana* that was added to the culture medium of the tank, was as used as inoculum. The amount of inoculum added was 0.174 g of dry matter per liter of culture medium (20.88 g for 120 liters), which resulted in an initial optical density measurement of 0.541 at a wavelength of 580 nm. The tank (11) incorporated therein a submerged pump (12) to drive the culture medium with microalgae to the perforated pipe which discharged it on the upper part of the panel. The culture medium with microalgae exiting through the perforated tube moved down through the sheets of PVC mesh forming the side walls of the photobioreactor (5) and was collected in the lower channel (8) which rechanneled it to tank (11) to be pumped again to the perforated PVC pipe in a cyclic operation. The system was maintained in a natural light and darkness cycle. Through the outer connection of the porous pipe located inside the reactor, the gasoline engine exhaust gas was injected at a rate of 1 liter of exhaust gas per minute, its mean CO₂ content being of the order of 8% by volume, which in normal conditions would provide approximately 9.8 g of CO₂/hour. The example described corresponds to a period of 12 hours whereby 117.6 g of CO₂ were provided to the culture daily, the carbon content of which would be 32 g.

Due to the evaporation occurring in the panels, after culturing for 5 days the volume of the liquid of the tank had changed from 120 to 30 liters and the amount of microalgae contained in said medium was 234.12 g (expressed as dry matter) which involved an increase of 213.12 g in 5 days (mean increase of 42.6 g/day). From the final volume (30 L), the concentration of which was 7.8 g/liter of dry matter of microalgae, 27 liters were removed and the rest was left to be used as an inoculum for the next culture (23.4 g), the remaining volume being topped up to 120 liters with freshly prepared culture medium. The cycle was repeated for another 5 days and so on and so forth. The 27 liters of concentrated algae culture were concentrated through the centrifugation of 120 to 30 liters (a 75% reduction). The 213.12 g of dry matter of microalgae produced in 5 days had a carbon content of 85.25 g (40% dry matter) and the CO₂ engine exhaust supplied in 5 days had a carbon content of 32 x 5 =160 g, therefore the CO₂ fixation rate with respect to the CO₂ supplied was 53.3%.

### Example 3:_Photobioreactor made up of several modules connected in parallel

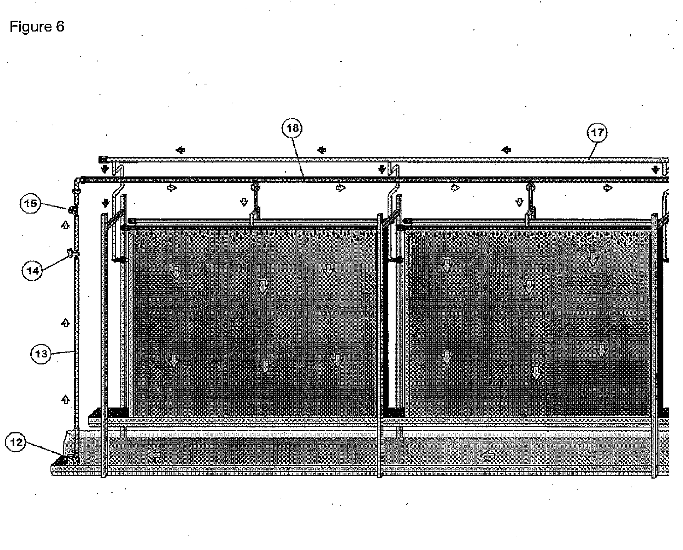

Three units of the photobioreactor of Example 1 were combined in a linear manner using the same collection channel for all of them (Figure 6). The CO₂-enriched air was supplied through a main pipe (17) from which connections for each unit of photobioreactor branched out. The culture medium with microalgae driven by the pump (12) submerged in the tank (11) was distributed to each unit of photobioreactor by means of a general pipe (18) from which the connections for each unit exited. In other words, the connections of each unit with the gas supply lines and supply lines for the culture with microalgae were made "in parallel".

### Brief Description of the Drawings

Figure 1 shows the supporting frame of the photobioreactor panel
   1. Rack of the frame
   2. Porous pipe for supplying gases into the chamber of the photobioreactor.
   3. Valve and conduit for the entrance of gases to the porous pipe
   4. Support for the porous pipe inside the chamber of the photobioreactor
Figure 2 shows the frame of the photobioreactor covered on both sides by the sheets of mesh
   5. Sheet of light mosquito net-like mesh covering both sides of the frame, leaving an air chamber between them.
Figure 3 shows the frame of the photobioreactor covered on both sides by sheets of mesh and by a sheet of fabric.
   6. Sheet of fabric placed on the sheet of mesh of the photobioreactor.
   7. Angular battens for securing the sheets of fabric to the frame of the photobioreactor.
Figure 4 shows the supporting structure of a panel of the photobioreactor.
   8. Channel on which the frame of the photobioreactor rests acting as a receiver of the microalgae culture flow moving down the side sheets of the panels.
   9. Sides supports for the panels and channel.
   10. Corrugated discharge pipe for discharging culture medium with microalgae on the upper part of the frame of the photobioreactor.
   11. System of collector tank for collecting the culture medium with microalgae.
   12. Circulation pump for circulating the culture medium with microalgae.
   13. Upward pipe for the culture medium with microalgae.
   14. Flow regulating valve for regulating the flow of culture medium with microalgae.
   15. Flow indicator
Figure 5 shows the modular unit of a photobioreactor.
   16. Gas inlet conduit connecting with the porous pipe inside the chamber of the photobioreactor, containing a flow rate regulating valve.
Figure 6 shows an overall view of a photobioreactor with 3 modules.
   17. General gas conduit for the gases which will be introduced into the chambers of each unit of photobioreactor.
   18. General pipe for channeling the culture medium with microalgae which is distributed to each panel through the respective perforated pipes located above each frame.

## Claims

1. A laminar photobioreactor for the production of microalgae, comprising:
- a panel formed by a rack or frame (1) covered at both sides by at least one sheet of mesh (5) through which a microalgae-containing liquid medium flows downwards, and
- an inner chamber between said sheets of mesh (5) and the walls of the rack or frame (1), air-tight by means of hydraulic closure.

2. The photobioreactor according to claim 1, wherein said sheet of mesh (5) is made of synthetic material, glass fiber, natural fiber, metallic material, or combinations thereof.

3. The photobioreactor according to claim 1 or 2, wherein said sheet of mesh (5) is supplemented by at least one sheet of fabric (6).

4. The photobioreactor according to claim 3, wherein said sheet of fabric (6) is made of synthetic material, natural fibers, or combinations thereof.

5. The photobioreactor according to claim 4, wherein said synthetic material is selected from the group consisting of PVC, polystyrene and polypropylene.

6. The photobioreactor according to any of claims 1 to 5 having a transparent plastic covering on said sheets at both sides.

7. The photobioreactor according to any of the preceding claims, wherein the lower edges of said sheets (5, 6) discharge the microalgae-containing liquid medium to a channel (8) communicating with a tank system (11) located in the lower part of the photobioreactor.

8. The photobioreactor according to claim 7, wherein the liquid medium is recirculated to the upper part of the frame (1) from said tank system (11).

9. The photobioreactor according to one of claims 7 or 8, wherein said tank system (11) includes a decanting tank allowing the sedimentation of the microalgae.

10. The photobioreactor according to claim 9, wherein said sedimentation occurs in the presence of flocculants.

11. The photobioreactor according to any of claims 1 to 10, wherein a gas is introduced into said chamber.

12. The photobioreactor according to claim 11, wherein said gas is isolated from direct solar radiation.

13. The photobioreactor according to claim 11 or 12, wherein said gas is air, carbon dioxide, combustion gases, or the mixtures thereof.

14. A set of at least two photobioreactors according to any of the preceding claims connected in parallel in a modular structure.
